# NEUE EUROPÄISCHE PATENTSCHRIFT

(11) **EP 3 089 711 B2**
(45) Veröffentlichungstag und Bekanntmachung des Hinweises auf die Entscheidung über den Einspruch: **03.01.2024**
(45) Hinweis auf die Patenterteilung: 06.12.2017
(21) Anmeldenummer: 14823911.4
(22) Anmeldetag: 31.12.2014
(51) Int. Cl.: A61F 2/64, A61F 2/50

(54) **PROTHESENKNIEGELENK**
PROSTHETIC KNEE JOINT
ARTICULATION DE GENOU PROTHÉTIQUE

(30) Priorität: 03.01.2014 DE 102014000020
(43) Veröffentlichungstag der Anmeldung: 09.11.2016
(73) Patentinhaber: Otto Bock HealthCare GmbH, 37115 Duderstadt (DE)
(72) Erfinder: BOITEN, Herman, 37085 Göttingen (DE)
(74) Vertreter: Stornebel, Kai
(86) Internationale Anmeldenummer: PCT/EP2014/003483
(87) Internationale Veröffentlichungsnummer: WO 2015/101417

(56) Entgegenhaltungen:
- EP-A1- 0 713 689
- DE-A1-102009 053 128
- DE-A1-102009 053 128
- US-A- 5 645 590
- US-A- 5 728 173
- US-A- 6 086 616
- US-A1- 2013 085 580
- US-B1- 6 706 074
- US-B1- 6 808 540
- US-B1- 6 808 540

## Beschreibung

Die Erfindung betrifft ein Prothesenkniegelenk mit einem Oberteil, einem Unterteil, das verschwenkbar zu dem Oberteil angeordnet ist, einer an dem Oberteil angeordneten Befestigungseinrichtung für ein proximales Prothesenelement, einer an dem Unterteil angeordneten Befestigungseinrichtung für ein distales Prothesenelement und einem viergliedrigen Gelenksystem mit vier gelenkig aneinander befestigten Gelenkgliedern, die jeweils um eine Schwenkachse zueinander verschwenkbar sind, wobei das Oberteil an dem Gelenksystem angeordnet ist.

Die EP 0 713 689 A1 zeigt ein Prothesenkniegelenk mit einem Oberteil und einem gelenkig daran befestigten Unterteil. Die Kopplung von Oberteil und Unterteil erfolgt über eine mehrgliedrige, kinematische Gelenkkette mit mindestens vier Gelenkgliedern, in der die jeweils miteinander verbundenen Glieder eine gemeinsame Drehachse aufweisen. Die Drehachsen verlaufen im Wesentlichen parallel zueinander, wobei in der Ausgangsposition des mehrgliedrigen Gelenksystems ein Gelenkglied gegenüber den mit ihm verbundenen anderen Gliedern zwei Bewegungen ausführen kann, wobei wenigstens eine Bewegung nach deren Einleitung die andere mögliche Bewegung zumindest überwiegend sperrt. Mit einer solchen Vorrichtung ist es möglich, eine Standphasenflexion zu ermöglich, ohne eine Einbeugung des Prothesenkniegelenkes komplett zu sperren.

Problematisch bei Prothesenkniegelenken mit längenveränderlichen Gelenkgliedern sind der relativ komplexe Aufbau und die Möglichkeit, dass eine geometrische Sperre des Gelenksystems beim Einsinken während der Standphasenbeugung auftritt. Eine geometrische Sperre eines Prothesenkniegelenkes ist beispielsweise in der US-A-5,314,498 beschrieben, bei dem die Funktionsweise eines natürlichen Kniegelenks durch einen polyzentrischen Aufbau nachgeahmt werden und auf eine einfache Art und Weise eine automatische geometrische Verriegelung erreicht werden soll.

Die US 6,808,540 B1 offenbart ein Prothesenkniegelenk, bei dem das Einbeugen des Gelenksystems, das aus vier aneinander angeordneten Lenkern besteht, gegen eine durch ein hydraulisches Element aufgebrachte Kraft erfolgt. Aus der DE 10 2009 053 128 A1 ist ein Fünflenker-Kniegelenk bekannt, bei dem wenigstens ein Gelenkglied längenveränderbar ausgestattet ist.

Aufgabe der vorliegenden Erfindung ist es, ein Prothesenkniegelenk bereitzustellen, das eine einfache Anordnung der Komponenten aufweist und gewährleistet, dass auch während der Standphasenbeugung ein Einbeugen des Prothesenkniegelenkes grundsätzlich möglich ist.

Erfindungsgemäß wird diese Aufgabe durch ein Prothesenkniegelenk mit den Merkmalen des Hauptanspruches gelöst. Vorteilhafte Ausgestaltungen und Weiterbildungen der Erfindung sind in den Unteransprüchen, der Beschreibung sowie den Figuren offenbart.

Das erfindungsgemäße Prothesenkniegelenk mit einem Oberteil, einem Unterteil, das verschwenkbar zu dem Oberteil daran befestigt ist, einer an dem Oberteil angeordneten Befestigungseinrichtung für ein proximales Prothesenelement, einer an dem Unterteil angeordneten Befestigungseinrichtung für ein distales Prothesenkniegelenk und einem viergliedrigen Gelenksystem mit vier gelenkig aneinander befestigten längenunveränderlichen Gelenkgliedern, dem jeweils eine Schwenkachse zueinander verschwenkbar sind, wobei das Oberteil an dem Gelenksystem angeordnet ist, sieht vor, dass das Gelenksystem aus einer Ausgangsstellung entgegen einer Federkraft während der Standphasenbeugung verschwenkbar an dem Unterteil gelagert ist, wobei die Stellung der Gelenkglieder zueinander unverändert bleibt und die Wirklinie der Federkraft so ausgerichtet ist, dass ein gegen die Einbeugung des Gelenksystem wirkendes Moment vorliegt, insbesondere auch bei einem gegenüber der Ausgangsstellung verschwenkten Gelenksystem oder einem extendierten Gelenksystem, dass gegen den Streckanschlag anliegt und in seiner Gesamtheit gegenüber dem Unterteil aufgrund der bei der Standphasenflexion auftretenden Kräfte verlagert ist. Zusätzlich weist das Gelenksystem eines erfindungsgemäßen Prothesenknieqelenks ein posteriores Gelenkglied auf, das über eine distale Schwenkachse hinaus verlängert ist und in der Verlängerung eine proximale Schwenkachse für ein Federelement (A) aufweist. Durch die Ausrichtung der Wirklinie der Federkraft durch eine entsprechende Anordnung und Orientierung der Gelenkpunkte, beispielsweise eines Federelementes, das als längenveränderliches Gelenkglied eines Mehrlenkersystems ausgebildet ist, ist es möglich, dass bei einer Standphasenbeugung ein sicherndes Moment auf das Gelenksystem ausübt, ohne dass es dabei zu einer Änderung der geometrischen Struktur und der Zuordnung der Gelenkglieder in dem Gelenksystem kommt. Das Gelenksystem wird insgesamt verschwenkt, ohne dass es zu einer Einbeugung des Prothesenkniegelenkes durch eine Verlagerung der Gelenkglieder des Gelenksystems zueinander kommt. Dadurch ist es möglich, dass trotz eines bei der Standphasenbeugung zusätzlichen Momentes entgegen einer Einbeugung des Gelenksystems die grundsätzliche Möglichkeit besteht, dass bei Aufbringen eines ausreichend hohen Beugemomentes, z B. des Hüftbeugemomentes, das Prothesenkniegelenk einbeugt, so dass eine geometrische Blockade, der sogenannte "geometric lock" nicht auftritt. Dies wird insbesondere dadurch erreicht, dass die Gelenkglieder des Gelenksystems längenunveränderlich ausgebildet sind und als System in ihrer Stellung zueinander unverändert insgesamt schwenkbar an dem Unterteil angeordnet sind.

Eine Weiterbildung der Erfindung sieht vor, dass mit zunehmendem Verschwenkwinkel des Gelenksystems gegen das Unterteil bei einer Standphasenbeugung das Moment erhöht wird, das gegen eine Einbeugung des Gelenksystems wirkt. Bei einem zunehmenden Verschwenkwinkel wird neben der sich vergrößernden Federkraft auch der wirksame Hebelarm vergrößert, wodurch sich eine doppelte Erhöhung des Momentes ergibt, so dass bei einer maximalen Standphaseneinbeugung ein maximal wirksames Moment gegen ein Einbeugen des Prothesenkniegelenkes durch eine Veränderung der Positionen der Gelenkglieder zueinander vorliegt. Das Oberteil und das Unterteil werden zwar durch das Einsinken in Richtung entgegen der Federkraft relativ zueinander verlagert, dies ist jedoch kein Einbeugen des Prothesenkniegelenks durch eine Veränderung der Orientierung der Gelenkglieder des Gelenksystems zueinander.

Eine Weiterbildung der Erfindung sieht vor, dass das Gelenksystem an einem Mehrlenkersystem gelagert ist, dessen Schwenkachsen nicht mit den Schwenkachsen des Gelenksystems zusammenfallen. Dies führt zu einer Entzerrung der Lagerstellen der jeweiligen Systeme, also des Gelenksystems und des Mehrlenkersystems, so dass keine Lagerstellen nebeneinander liegen und dadurch die Lagerbreiten ausreichend stabil dimensioniert werden können.

Das Gelenksystem kann einen mechanischen Streckanschlag aufweisen, gegen den das Gelenksystem in der gestreckten, extendierten Stellung anliegt. Eine Standphasenflexion wird in der Regel bei einem gestreckten Prothesenkniegelenk auftreten, so dass bei dem sogenannten "heel strike" das Gelenksystem an dem mechanischen Streckanschlag anliegt. Dies kann beispielsweise dadurch geschehen, dass eines der Gelenkglieder an dem mechanischen Streckanschlag anliegt und dadurch eine Weiterverlagerung des Gelenkgliedes in Extensionsrichtung und dadurch eine entsprechende Relativverlagerung der Gelenkglieder zueinander verhindert wird.

Das Gelenksystem kann ein distales Gelenkglied aufweisen, das eine weitere anteriore Schwenkachse aufweist, die distal zu der anterioren Schwenkachse des Gelenksystems angeordnet ist und um die das Gelenksystem schwenkbar an dem Unterteil gelagert ist. Das distale Gelenkglied weist somit zwei Funktionen auf, einmal die Verbindung zweier Gelenkglieder des Gelenksystems, nämlich des anterioren und des posterioren Gelenkgliedes, und darüber hinaus die Ausbildung einer Schwenkachse bzw. einer Lagerstelle für eine Schwenkachse, um das Gelenksystem mit dem Unterteil zu verbinden und dadurch ein Mehrlenkersystem zur verschwenkbaren Lagerung des Gelenksystems an dem Unterteil bereitzustellen.

Das Gelenksystem kann darüber hinaus ein posteriores Gelenkglied aufweisen, das eine proximale und eine distale Schwenkachse aufweist, wobei des posteriore Gelenkglied erfindungsgemäß über die distale Schwenkachse hinaus verlängert ist und in der Verlängerung eine proximale Schwenkachse für ein Federelement aufweist, das Teil des Mehrlenkersystems ist. Das posteriore Gelenkglied stellt somit ebenso eine Lagerstelle des Mehrlenkersystems zur Verfügung, und zwar im Bereich der distalen Verlängerung über die distale posteriore Schwenkachse des Gelenksystems hinaus.

Das Federelement kann eine distale Lagerstelle aufweisen, die an dem Unterteil angeordnet sein kann, wobei die Verbindungslinie zwischen der distalen Lagerungsstelle des Federelementes und der proximalen Schwenkachse des Mehrlenkersystems anterior zur distalen Schwenkachse des posterioren Gelenkgliedes des Gelenksystems verläuft. Dadurch wird sichergestellt, dass bei Aufbringen einer Kraft auf das Federelement bei der Standphasenbeugung über die geometrische Zuordnung und die Wirklinie der Krafteinleitungspunkte ein Moment erzeugt wird, das gegen ein Einbeugen des Prothesenkniegelenkes, also eine Verschwenkung des Gelenksystems und dadurch einer Verlagerung und Einbeugung des Oberteils relativ zu dem Unterteil, entgegenwirkt.

Das Federelement, das das Gelenksystem gegenüber dem Unterteil abstützt und ein Einsinken in der Standphase ermöglicht, kann nicht nur als ein reines Federelement, sondern auch als ein Feder-Dämpfer-Element oder Feder-Dämpfer-System ausgebildet sein, um eine Rückstellkraft gegen ein Einsinken des Gelenksystems bereitzustellen. Bei einer Ausgestaltung des Federelementes mit einer Dämpferkomponente kann die Dämpfung über einen Fluiddämpfer oder einen Elastomerdämpfer bereitgestellt werden. Der Elastomerdämpfer kann ein Elastomerelement aufweisen oder aus einem Elastomerelement bestehen, das aus einem Kunststoffkomposit ausgebildet ist. Das Elastomerelement kann als Tubus ausgebildet sein oder zumindest einen Tubus aufweisen, wobei es möglich ist, dass mehrere Elastomerelemente in Reihe geschaltet angeordnet sind.

An einem distalen Gelenkglied des Gelenksystems kann ein Federelement angeordnet sein, dass sich an dem proximalen Gelenkglied des Gelenksystems abstützt. Über dieses Federelement ist es möglich, das Verhalten des Prothesenkniegelenkes bei der normalen Einbeugung, also während der Schwungphase oder beim Einbeugen des Prothesenkniegelenkes im Bereich der terminalen Standphase, zu beeinflussen. Das Federelement des Gelenksystems kann analog zu dem Federelement zwischen dem Gelenksystem und dem Unterteil nicht nur als ein reines Federelement, sondern auch als ein Feder-Dämpfer-Element oder Feder-Dämpfer-System ausgebildet sein, das eine Rückstellkraft bereitstellt, mit der das Gelenksystem in eine Ausgangsposition, insbesondere die vollständig extendierte Position, bewegt wird, wenn keine anderen Kräfte auf das Prothesenkniegelenk wirken. Bei einer Ausgestaltung des Federelementes mit einer Dämpferkomponente kann die Dämpfung über einen Fluiddämpfer oder einen Elastomerdämpfer bereitgestellt werden. Der Elastomerdämpfer kann ein Elastomerelement aufweisen oder aus einem Elastomerelement bestehen, das aus einem Kunststoffkomposit ausgebildet ist. Das Elastomerelement kann als Tubus ausgebildet sein oder zumindest einen Tubus aufweisen, wobei es möglich ist, dass mehrere Elastomerelemente in Reihe geschaltet angeordnet sind.

Das Gelenksystem ist vorteilhafterweise über ein Federelement an dem Unterteil abgestützt, wobei die Befestigungsstellen des Federelementes nicht mit den Schwenkachsen des Gelenksystems zusammenfallen, wodurch eine Entflechtung der Lagerstellen stattfindet. Keine Lagerstelle des Gelenksystems fällt somit mit einer Lagerstelle des Mehrlenkersystems zusammen, so dass sämtliche Lagerstellen oder Verbindungsstellen der Gelenkglieder sowohl des Gelenksystems als auch des Mehrlenkersystems separat ausgebildet und ausreichend stark dimensioniert werden können. Die Gelenkglieder des Gelenksystems sind vorzugsweise längenunveränderlich, während zumindest eines der Gelenkglieder des Mehrlenkersystems längenveränderlich ist, so dass eine Veränderung der Geometrie des Mehrlenkersystems nicht nur durch eine Rotation zweier aneinander befestigter Gelenkglieder, sondern auch durch eine Längenveränderung der Gelenkglieder und einer Veränderung des Abstandes der Lagerstellen eines Gelenkgliedes zueinander stattfindet.

Sofern ein Federelement vorgesehen ist, kann dieses als ein komprimierbares Elastomerelement ausgebildet sein, wodurch es möglich ist, bei einer geringen Bauhöhe und bei einem geringen Bauraum hohe Federkräfte aufzubringen, darüber hinaus ist ein solches Elastomerelement wartungsfrei, geräuscharm und sehr leicht. Das Federelement kann sowohl im Gelenksystem als auch im Mehrlenkersystem angeordnet sein.

Das Federelement kann hinsichtlich der Rückstellkraft einstellbar ausgestaltet sein, um eine Anpassung der Prothese an den jeweiligen Patienten zu ermöglichen. Die Einstellbarkeit kann für ein oder beide Federelemente gegeben sein. Das jeweilige Federelement kann vorgespannt sein und über die Vorkompression den Widerstand gegen ein Einbeugen oder den Widerstand gegen ein Einsinken des Gelenksystems beeinflussen. Bei dem Federelement zwischen dem Gelenksystem und dem Unterteil als Teil des Mehrlenkersystems kann der Grad der Vorkompression, der einstellbar und veränderbar ist, eine Anpassung an das Eigengewicht des Patienten oder an die beabsichtigte Belastung ermöglichen.

Nachfolgend wird ein Ausführungsbeispiel der Erfindung anhand der beigefügten Figuren näher erläutert. Es zeigen:
- Figur 1 -: eine schematische Darstellung eines Prothesenkniegelenkes in vollständig extendierter Stellung; sowie
- Figur 2 -: ein Prothesenkniegelenk mit einem relativ zum Unterteil verlagerten Gelenksystem.

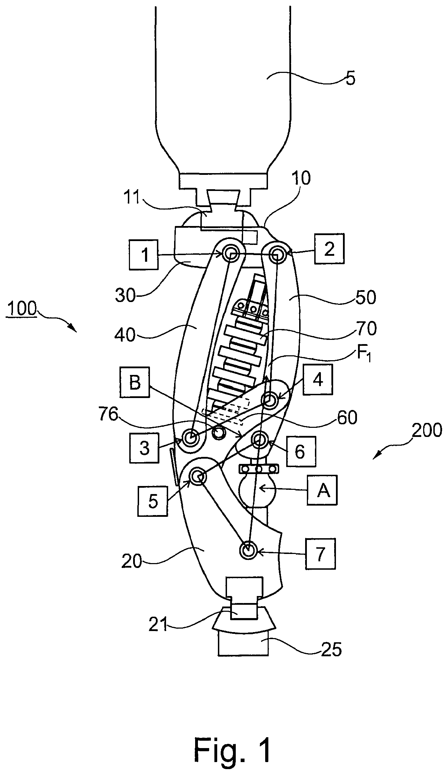

Das in Figur 1 gezeigte Ausführungsbeispiel eines Prothesenkniegelenkes weist ein Oberteil 10 auf, an dem eine Befestigungseinrichtung 11 in Gestalt eines Anschlusspylons für eine distale Prothesenkomponente 5 vorgesehen ist, beispielsweise zur Befestigung des Prothesenkniegelenkes an einem Oberschenkelrohr oder an einem Oberschenkelschaft. Die Befestigungseinrichtung 11 kann in dem Oberteil 10 eingeschraubt oder einstückig daran ausgebildet sein. An dem Oberteil 10 kann ein erstes Gelenkglied 30 eines viergliedrigen Gelenksystems 100 angeordnet oder ausgebildet sein. Das proximale, im Wesentlichen horizontal orientierte erste Gelenkglied 30 weist eine anteriore Schwenkachse 1 und eine posteriore Schwenkachse 2 auf. An der anterioren Schwenkachse 1 ist ein anteriores Gelenkglied 40 schwenkbar gelagert, an der posterioren Schwenkachse 2 ist ein posteriores Gelenkglied 50 schwenkbar gelagert. Über die Schwenkachse 1 ist somit das proximale Ende des anterioren Gelenkgliedes 40 und über die Schwenkachse 2 das proximale Ende des posterioren Gelenkgliedes 50 schwenkbar mit dem Oberteil 10 bzw. dem proximalen Gelenkglied 30 verbunden.

Das distale Ende des anterioren Gelenkgliedes 40 ist an einer Schwenkachse 3 schwenkbar an einem distalen Gelenkglied 60 gelagert. Die Schwenkachse 3 ist an dem anterioren Ende des distalen Gelenkgliedes 60 angeordnet. An dem posterioren Ende des distalen Gelenkgliedes 60 ist eine vierte Schwenkachse 4 angeordnet, an dem die proximale Lagerstelle des posterioren Gelenkgliedes 50 des viergliedrigen Gelenksystems 100 gelagert ist. Das Gelenksystem 100 wird somit durch die vier Schwenkachsen 1, 2, 3 und 4 der vier Gelenkglieder 30, 40, 50 und 60 gebildet. Die Gelenkglieder 30, 40, 50, 60 sind starr ausgebildet, so dass der Abstand der Schwenkachsen 1, 2, 3, 4 innerhalb der Gelenkglieder 30, 40, 50, 60 unveränderlich bleibt, durch eine Rotation der Gelenkglieder zueinander ist es möglich, dass das Oberteil 10 zum Einbeugen des Prothesenkniegelenkes, beispielsweise während einer Schwungphase, eingebeugt wird. Die Einbeugung kann nahezu 180° betragen. Das Oberteil 10 wird relativ zu einem Unterteil 20 durch die Verlagerung der Gelenkglieder 30, 40, 50, 60 des viergliedrigen Gelenksystems 100 eingebeugt. Das Unterteil 20 weist analog zu dem Oberteil 10 eine Befestigungseinrichtung 21 auf, die zur Befestigung eines distalen Prothesenelementes 25, beispielsweise eines Prothesenfußes oder eines dargestellten Unterschenkelrohres dient.

Das distale Gelenkglied 60 weist neben der distalen Schwenkachse 3 eine weitere Schwenkachse 5 auf, die distal zu der Schwenkachse 3 beabstandet ist, so dass das gesamte Gelenksystem 100 um die Schwenkachse 5 relativ zu dem Unterteil 20 verschwenkt werden kann, auch wenn keine Einbeugung des Oberteils 10 durch eine Verlagerung der Gelenkglieder 30, 40, 50, 60 des Gelenksystems 100 zueinander stattfindet. Dazu ist vorgesehen, dass distal zu der distalen, posterioren Schwenkachse 4 eine Verlängerung des posterioren Gelenkgliedes 50 ausgebildet ist, an der eine distale, posteriore Schwenkachse 6 angeordnet ist, an der ein Federelement A befestigt ist, das sich an dem Unterteil 20 an einer unteren Schwenkachse 7 abstützt. Durch die Schwenkachsen 5, 6, 7 wird somit ein Mehrlenkersystem ausgebildet, das durch das Federelement A, die Verbindung zwischen den an dem Unterteil 20 angeordneten Gelenkachsen 5, 7 sowie der Verbindung zwischen der proximalen Schwenkachse 5 am Unterteil 20 und der distalen Verlängerung des posterioren Gelenkgliedes 50 der dortigen Schwenkachse 6 ausbildet. Die Länge des posterioren Gelenkgliedes 50, das zwischen den Schwenkachsen 6 und 7 liegt, des Mehrlenkersystems 200 ist veränderlich.

Das Federelement A ist vorzugsweise als ein Elastomerelement ausgebildet und ermöglicht, dass das Gelenksystem 100 entgegen einer Federkraft in einer Ausgangsposition gehalten wird. Das Prothesenkniegelenk ist in dieser Ausgangsposition dargestellt, in der sowohl das Federelement A das Gelenksystem 100 in seine Ausgangsstellung drückt und in der das Gelenksystem 100 maximal in anteriore Richtung verlagert ist.

In der dargestellten Ausgangsstellung befindet sich das Prothesenkniegelenk in einer maximal extendierten Stellung, eine Einbeugung ist weder durch eine Kompression des Federelementes A noch durch eine Verlagerung der Gelenkglieder 30, 40, 50, 60 zueinander gegeben. Das Prothesenkniegelenk ist maximal extendiert und liegt an einem Streckanschlag B an, der im dargestellten Ausführungsbeispiel durch einen Vorsprung an der distalen Verlängerung des posterioren Gelenkgliedes 50 ausgebildet ist, der an der Unterseite des distalen Gelenkgliedes 60 anliegt. Grundsätzlich ist es auch möglich, den Streckanschlag B anders zu positionieren.

Innerhalb des Gelenksystems 100 ist ein weiteres Federelement 70 aus komprimierbaren Elastomerelementen angeordnet, das an dem proximalen Ende an dem Oberteil 10 und an dem distalen Ende an dem distalen Gelenkglied 60 gelagert ist. Die distale Lagerstelle 76 befindet sich zwischen den Schwenkachsen 3 und 4 an dem distalen Gelenkteil 60. Das proximale Ende des Federelementes 70 ist zwischen den proximalen Schwenkachsen 1, 2 angeordnet. Im dargestellten Ausführungsbeispiel fallen die Befestigungsstellen des Federelementes nicht mit einer der Schwenkachsen 1, 2, 3, 4 zusammen, grundsätzlich ist es auch möglich, dass eine oder beide Lagerstellen des Federelementes 70 mit einer bzw. zwei Schwenkachsen zusammenfallen. Über das Federelement 70 ist es möglich, das Einbeugen des Prothesenkniegelenkes während der Schwungphase oder der terminalen Standphase zu beeinflussen.

Die Funktionsweise des Prothesenkniegelenkes sieht vor, dass z.B. am Ende der Schwungphase, bei der ein maximal extendiertes Prothesenkniegelenk vorliegt, der sogenannte "heel strike" oder Fersenstoß erfolgt, bei dem die Ferse mit dem extendierten Prothesenkniegelenk auf den Boden aufsetzt. Um diesen Fersenstoß abzufangen, verschwenkt der obere Teil des Prothesenkniegelenkes mit dem viergliedrigen Gelenksystem 100 komplett in der extendierten Stellung, in der der Streckanschlag B an dem distalen Gelenkglied 60 anliegt, um die Schwenkachse 5 und komprimiert das Federelement A. Eine Verschwenkung um die anteriore Schwenkachse 5 des Mehrlenkersystems 200 hat nur eine geringe Verlagerung des Oberteils 10 zu dem Unterteil 20 zur Folge, ein Beugewinkel von 5° ist üblich. Bereits in der Ausgangsstellung, wie sie in der Figur 1 dargestellt ist, liegt die Kraftwirkungslinie F , also die Verbindungslinie zwischen den Lagerungsstellen 6, 7 des Federelementes A, vor bzw. anterior der distalen, posterioren Schwenkachse 4 des viergliedrigen Gelenksystems 100, so dass bei einer Verschwenkung um die Schwenkachse 5 eine Kraft ausgeübt wird, die das posteriore Gelenkglied 50 gegen das distale Gelenkglied 60 drückt. Dadurch entsteht ein Moment um die distale, posteriore Schwenkachse, so dass eine Erhöhung des wirksamen Momentes gegen eine Einbeugung des Gelenksystems 100 erfolgt. Bei zunehmender Standphasenbeugung, also bei einem zunehmendem Verschwenkwinkel um die Schwenkachse 5 des Mehrlenkersystems 200 wandert die Wirkungslinie F der Federkraft in anteriore Richtung, wodurch sich der Hebel um die distale, posteriore Schwenkachse 4 vergrößert, so dass ein sich vergrößerndes Moment bei einem zunehmenden Verschwenkwinkel des Gelenksystems 100 um die Schwenkachse 5 einstellt. Dadurch wird eine Vergrößerung der Sicherheit des Prothesenkniegelenkes gegen ein unbeabsichtigtes Einbeugen durch eine Relativbewegung der Gelenkglieder 30, 40, 50, 60 zueinander durch die Verschwenkung um die Schwenkachse 1, 2, 3, 4 bereitgestellt. Diese Erhöhung der Sicherheit bei einer zunehmenden Standphasenbeugung, insbesondere bei einer Fersenbelastung, führt jedoch nicht zu einer geometrischen Verriegelung des Prothesenkniegelenkes, vielmehr kann es durch Aufbringen eines Hüftbeugemomentes durch Veränderung der Geometrie des Gelenksystems 100 weiterhin eingebeugt werden, so dass der Sturz über ein gestrecktes Bein wirksam vermieden werden kann.

Das erfindungsgemäße Prothesenkniegelenk vermeidet, dass die Lagerstellen der einzelnen Komponenten zusammenfallen, so dass eine stabile und einfache Konstruktion des Prothesenkniegelenkes erreicht werden kann. Durch den anterioren Verlauf der Kraftwirkungslinie F des Mehrlenkersystems ist es möglich, während der Standphasenbeugung, insbesondere während des "heel strikes", eine erhöhte Sicherheit bereitzustellen. Ein Einbeugen durch Aufbringen eines Hüftbeugemomentes, was beispielsweise auch am Ende der Standphase erfolgt, ist ohne Weiteres möglich.

An der Befestigungseinrichtung 11 des Oberteils 10 ist als oberes Anschlussmittel ein Prothesenschaft 5 zur Aufnahme eines Amputationsstumpfes als proximales Prothesenelement angedeutet, als distales Prothesenelement 25 ist ein Unterschenkelrohr an dem Unterteil 20 an der distalen Befestigungseinrichtung 21 angeordnet.

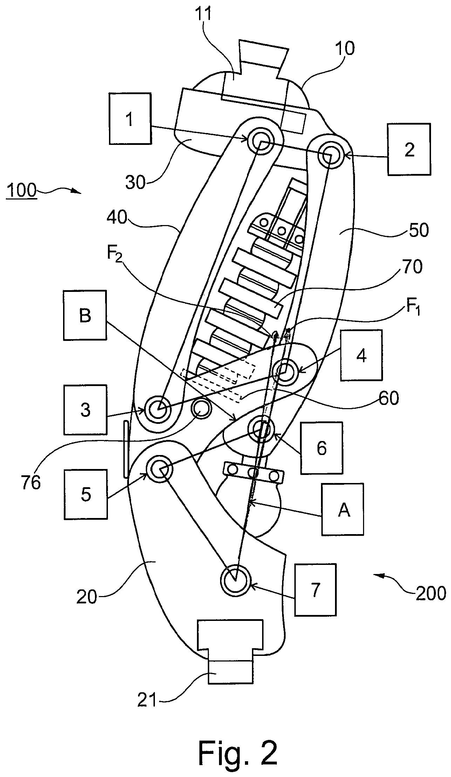

In der Figur 2 ist das in der Figur 1 dargestellte Prothesenkniegelenk in einem Zustand gezeigt, in der das in sich unveränderte Gelenksystem 100 um die distal zu der anterioren, distalen Schwenkachse 3 an dem distalen Gelenkglied 60 angeordnete Schwenkachse 5 verschwenkt ist. Das Gelenksystem 100 mit den Schwenkachsen 1, 2, 3, 4 und den zueinander im Verhältnis zur Figur 1 unveränderten Gelenkgliedern 30, 40, 50, 60 wurde um die Schwenkachse 5 in Uhrzeigerrichtung verschwenkt. Das Verschwenken erfolgte aufgrund einer Längenveränderung des Federelementes A des Mehrlenkersystems 200, beispielsweise aufgrund des heel strikes oder während des Stehens bei einer Fersenbelastung. In der Figur 2 sind zwei Wirklinien F der Federkraft eingezeichnet, nämlich die ursprüngliche Wirklinie F1 gemäß der Figur 1 und die tatsächlich wirksame Wirklinie F2, die die Verlängerung der Verbindungslinie zwischen den distalen und proximalen posterioren Schwenkachsen 6 und 7 des Mehrlenkersystems 200 darstellt. In der Figur 2 ist zu erkennen, dass die wirksame Wirklinie F2 im Vergleich zu der ursprünglichen Wirklinie F1 in anteriorer Richtung bezogen auf die posteriore, distale Schwenkachse 4 des Gelenksystems 100 verlagert ist. Dadurch vergrößert sich der Hebelarm um die Schwenkachse 4 ebenso wie die Federkraft als solche, wodurch sich ein Moment ergibt, das einer Einbeugung des Prothesenkniegelenkes durch eine Veränderung der Zuordnung der Gelenkglieder 30, 40, 50, 60 des Gelenksystems 100 entgegenwirkt. Je größer die Verlagerung des Gelenksystems 100 um die Schwenkachse 5 in Uhrzeigerrichtung ist, desto größer ist die auf das Gelenksystem 100 über die distale Verlängerung des proximalen Gelenkgliedes 50 wirkende Federkraft und aufgrund der geometrischen Zuordnung desto größer ist der wirksame Hebelarm der Wirklinie F der Federkraft um die posteriore, distale Schwenkachse 4 und desto größer ist das gegen eine Einbeugung des Gelenksystems wirkende Moment.

## Patentansprüche

1. Prothesenkniegelenk mit
- einem Oberteil (10),
- einem Unterteil (20), das verschwenkbar zu dem Oberteil (10) daran angeordnet ist,
- einer an dem Oberteil (10) angeordneten Befestigungseinrichtung (11) für ein proximales Prothesenelement,
- einer an dem Unterteil (20) angeordneten Befestigungseinrichtung (21) für ein distales Prothesenelement,
- einem viergliederigen Gelenksystem (100) mit vier gelenkig aneinander befestigten längenunveränderlichen Gelenkgliedern (30, 40, 50, 60), die um jeweils eine Schwenkachse (1, 2, 3, 4) zueinander verschwenkbar sind, wobei das Oberteil (10) an dem Gelenksystem (100) angeordnet ist, **dadurch gekennzeichnet, dass** das Gelenksystem (100) aus einer Ausgangsstellung entgegen einer Federkraft während einer Standphasenflexion verschwenkbar an dem Unterteil (20) gelagert ist, wobei die Stellung der Gelenkglieder zueinander unverändert bleibt und die Wirklinie (F) der Federkraft so ausgerichtet ist, dass ein gegen eine Einbeugung des Gelenksystems (100) wirkendes Moment vorliegt,
- wobei das Gelenksystem (100) ein posteriores Gelenkglied (50) aufweist, das über eine distale Schwenkachse (4) hinaus verlängert ist und in der Verlängerung eine proximale Schwenkachse (6) für ein Federelement (A) aufweist.

2. Prothesenkniegelenk nach Anspruch 1, **dadurch gekennzeichnet, dass** bei einem zunehmenden Verschwenkwinkel des Gelenksystems (100) gegenüber dem Unterteil (20) das entgegen der Einbeugung wirksame Moment vergrößert wird.

3. Prothesenkniegelenk nach Anspruch 1 oder 2, **dadurch gekennzeichnet, dass** das Gelenksystem (100) an einem Mehrlenkersystem (200) gelagert ist, dessen Schwenkachsen (5, 6, 7) nicht mit den Schwenkachsen (1, 2, 3, 4) des Gelenksystems (100) zusammenfallen.

4. Prothesenkniegelenk nach einem der voranstehenden Ansprüche, **dadurch gekennzeichnet, dass** das Gelenksystem (100) einen mechanischen Streckanschlag (B) aufweist.

5. Prothesenkniegelenk nach einem der voranstehenden Ansprüche, **dadurch gekennzeichnet, dass** das Gelenksystem (100) ein distales Gelenkglied (60) aufweist, das um eine anteriore Schwenkachse (5), die distal zu einer anterioren Schwenkachse (3) des Gelenksystems (100) angeordnet ist, an dem Unterteil (20) gelagert ist.

6. Prothesenkniegelenk nach Anspruch 5, **dadurch gekennzeichnet, dass** das Federelement (A) eine distale Lagerstelle (7) aufweist und die Verbindungslinie zwischen der distalen Lagerstelle (7) und der proximalen Schwenkachse (6) anterior zu der distalen Schwenkachse (4) des posterioren Gelenkgliedes (50) verläuft.

7. Prothesenkniegelenk nach einem der voranstehenden Ansprüche, **dadurch gekennzeichnet, dass** an einem distalen Gelenkglied (60) des Gelenksystems (100) ein Federelement (70) angeordnet ist, die sich an einem proximalen Gelenkglied (30) des Gelenksystems (100) abstützt.

8. Prothesenkniegelenk nach einem der voranstehenden Ansprüche, **dadurch gekennzeichnet, dass** das Gelenksystem (100) über ein Federelement (A) an dem Unterteil (20) abgestützt ist und die Befestigungsstellen (6, 7) des Federelementes (A) nicht mit den Schwenkachsen (1, 2, 3, 4) des Gelenksystems (100) zusammenfallen.

9. Prothesenkniegelenk nach einem der Ansprüche 7 oder 8, **dadurch gekennzeichnet, dass** das Federelement (70, A) als ein komprimierbares Elastomerelement ausgebildet ist.

## Claims

1. Prosthetic knee joint comprising
- an upper part (10),
- a lower part (20), which is arranged on the upper part (10) in a manner pivotable thereto,
- a fastening device (11) arranged on the upper part (10) and provided for a proximal prosthetic element,
- a fastening device (21) arranged on the lower part (20) and provided for a distal prosthetic element,
- a four-member joint system (100) with four joint members (30, 40, 50, 60) which are unmodifiable in length and fastened to one another in an articulated manner and are pivotable with respect to one another about a respective pivot axis (1, 2, 3, 4), wherein the upper part (10) is arranged on the joint system (100), **characterized in that** the joint system (100) is mounted on the lower part (20) so as to be pivotable from a starting position counter to a spring force during a stance phase flexion, wherein the position of the joint members relative to each other remains unchanged and the action line (F) of the spring force is oriented such that a moment acting against a buckling of the joint system (100) is present,
- wherein the joint system (100) has a posterior joint member (50) which is continued beyond a distal pivot axis (4) and, in the continuation, has a proximal pivot axis (6) for a first spring element (A).

2. Prosthetic knee joint as claimed in claim 1, **characterized in that** the momentum acting counter to the buckling is increased as the angle of pivoting of the joint system (100) with respect to the lower part (20) increases.

3. Prosthetic knee joint as claimed in claim 1 or 2, **characterized in that** the joint system (100) is mounted on a multi-link system (200), of which the pivot axes (5, 6, 7) do not coincide with the pivot axes (1, 2, 3, 4) of the joint system (100).

4. Prosthetic knee joint as claimed in one of the preceding claims, **characterized in that** the joint system (100) has a mechanical extension stop (B).

5. Prosthetic knee joint as claimed in one of the preceding claims, **characterized in that** the joint system (100) has a distal joint member (60) which is mounted on the lower part (20) about an anterior pivot axis (5), which is arranged distally in relation to an anterior pivot axis (3) of the joint system (100).

6. Prosthetic knee joint as claimed in claim 5, **characterized in that** the first spring element (A) has a distal bearing point (7), and the connecting line between the distal bearing point (7) and the proximal pivot axis (6) extends anteriorly with respect to the distal pivot axis (4) of the posterior joint member (50).

7. Prosthetic knee joint as claimed in one of the preceding claims, **characterized in that** a second spring element (70) is arranged on a distal joint member (60) of the joint system (100) and is supported on a proximal joint member (30) of the joint system (100).

8. Prosthetic knee joint as claimed in one of the preceding claims, **characterized in that** the joint system (100) is supported on the lower part (20) via a first spring element (A), and the fastening points (6, 7) of the first spring element (A) do not coincide with the pivot axes (1, 2, 3, 4) of the joint system (100).

9. Prosthetic knee joint as claimed in either of claims 7 and 8, **characterized in that** the first and/or second spring element (70, A) is designed as a compressible elastomer element.

## Revendications

1. Articulation de genou prothétique, comportant
- une partie supérieure (10),
- une partie inférieure (20) qui est agencée sur la partie supérieure (10) en pouvant basculer par rapport à celle-ci,
- un moyen de fixation (11) agencé sur la partie supérieure (10) et destiné à un élément de prothèse proximal,
- un moyen de fixation (21) agencé sur la partie inférieure (20) et destiné à un élément de prothèse distal,
- un système d'articulation (100) à quatre membres, comportant quatre membres d'articulation (30, 40, 50, 60) fixés en articulation les uns aux autres et invariables vis-à-vis de leur longueur, qui sont aptes à basculer les uns par rapport aux autres chacun autour d'un axe de basculement (1, 2, 3, 4), dans laquelle la partie supérieure (10) est agencée sur le système d'articulation (100),
**caractérisée en ce que**
le système d'articulation (100) est monté sur la partie inférieure (20) avec faculté de basculement depuis une position de départ à l'encontre d'une force élastique pendant une flexion en phase stationnaire,
et la position mutuelle des membres d'articulation reste inchangée et la ligne d'action (F) de la force élastique est orientée de telle sorte qu'il y a un couple qui agit à l'encontre d'une flexion du système d'articulation (100),
- le système d'articulation (100) présentant un membre d'articulation postérieur (50) qui est prolongé au-delà d'un axe de basculement distal (4) et qui présente en prolongement un axe de basculement proximal (6) pour un élément élastique (A).

2. Articulation de genou prothétique selon la revendication 1,
**caractérisée en ce que** lors d'une augmentation de l'angle de basculement du système d'articulation (100) par rapport à la partie inférieure (20), le couple agissant à l'encontre d'une flexion est agrandi.

3. Articulation de genou prothétique selon la revendication 1 ou 2,
**caractérisée en ce que** le système d'articulation (100) est monté sur un système à bras multiples (200) dont les axes de basculement (5, 6, 7) ne coïncident pas avec les axes de basculement (1, 2, 3, 4) du système d'articulation (100).

4. Articulation de genou prothétique selon l'une des revendications précédentes,
**caractérisée en ce que** le système d'articulation (100) présente une butée d'extension mécanique (B).

5. Articulation de genou prothétique selon l'une des revendications précédentes,
**caractérisée en ce que** le système d'articulation (100) présente un membre d'articulation distal (60) qui est monté sur la partie inférieure (20) autour d'un axe de basculement antérieur (5) qui est agencé de façon distale par rapport à un axe de basculement antérieur (3) du système d'articulation (100).

6. Articulation de genou prothétique selon la revendication 5,
**caractérisée en ce que** l'élément élastique (A) comprend un emplacement de montage distal (7), et la ligne de liaison entre l'emplacement de montage distal (7) et l'axe de basculement proximal (6) s'étend de façon antérieure par rapport à l'axe de basculement distal (4) du membre d'articulation postérieur (50).

7. Articulation de genou prothétique selon l'une des revendications précédentes,
**caractérisée en ce qu'**un élément élastique (70) est agencé sur un membre d'articulation distal (60) du système d'articulation (100), qui s'appuie contre un membre d'articulation proximal (30) du système d'articulation (100).

8. Articulation de genou prothétique selon l'une des revendications précédentes,
**caractérisée en ce que** le système d'articulation (100) s'appuie sur la partie inférieure (20) par un élément élastique (A), et les emplacements de fixation (6, 7) de l'élément élastique (A) ne coïncident pas avec les axes de basculement (1, 2, 3, 4) du système d'articulation (100).

9. Articulation de genou prothétique selon l'une des revendications 7 ou 8, **caractérisée en ce que** l'élément élastique (70, A) est réalisé sous la forme d'un élément à base d'élastomère comprimable.
